# EUROPEAN PATENT SPECIFICATION

(11) **EP 2 367 548 B1**
(45) Date of publication and mention of the grant of the patent: **07.08.2013**
(21) Application number: 09795396.2
(22) Date of filing: 14.12.2009
(51) Int. Cl.: A61K 31/215, A61K 31/201, A61P 25/28

(54) **Medical food composition for treating neurodegenerative diseases**
Medizinische, Nahrungsmittelszusammensetzung zur Behandlung von neurodegenerativen Erkrankungen
Composition alimentaire médicale pour traiter les maladies neurodégénératives

(30) Priority: 15.12.2008 IT MI20082221
(43) Date of publication of application: 28.09.2011
(73) Proprietor: Sofedus S.r.l., 20124 Milano (IT)
(72) Inventor: PETRONI, Anna, I-20090 Segrate (IT); BANNI, Sebastiano, I-09126 Cagliari (IT); CAPPA, Marco, I-00124 Roma (IT)
(74) Representative: Asensio, Raffaella Consuelo
(86) International application number: PCT/EP2009/067070
(87) International publication number: WO 2010/069915

(56) References cited:
- WO-A-96/17621
- GOLOVKO AND MURPHY,: "uptake and metabolism." E.J.J.LIPID RES., 2006, XP008113554
- RUIZ ET AL.: "Glycerol trioleate/glycerol trierucate..." JOURNAL OF INHERITED METABOLIC DISEASE, vol. 19, no. 2, 1996, pages 188-192, XP008113547
- FA ET AL: "Incorporation and metabolism of c9,t11 and t10,c12 conjugated linoleic acid (CLA) isomers in rat brain" BIOCHIMICA AND BIOPHYSICA ACTA. MOLECULAR AND CELL BIOLOGY OFLIPIDS, ELSEVIER, AMSTERDAM, NL, vol. 1736, no. 1, 5 September 2005 (2005-09-05), pages 61-66, XP005046470 ISSN: 1388-1981
- PETRONI ANNA ET AL: "Metabolism of 8-iso-PGF2alpha and conjugated linoleic acid (CLA) in vivo and in X-Adrenoleukodystrophy fibroblasts" FASEB JOURNAL, vol. 21, no. 5, April 2007 (2007-04), page A23, XP008113532 & EXPERIMENTAL BIOLOGY 2007 ANNUAL MEETING; WASHINGTON, DC, USA; APRIL 28 -MAY 02, 2007 ISSN: 0892-6638
- IANNONE A ET AL: "Impairment of 8-iso-PGF2ALPHA isoprostane metabolism by dietary conjugated linoleic acid (CLA)" PROSTAGLANDINS LEUKOTRIENES AND ESSENTIAL FATTY ACIDS, CHURCHILL LIVINGSTONE, EDINBURGH, vol. 80, no. 5-6, 1 May 2009 (2009-05-01), pages 279-287, XP026148332 ISSN: 0952-3278 [retrieved on 2009-04-28]

## Description

### FIELD OF THE INVENTION

The present invention relates to a medical dietary composition for treating neurodegenerative diseases and in particular for treating adrenoleukodystrophy.

### PRIOR ART

Adrenoleukodystrophy (X-ALD, OMIM #300100) is a progressive neurodegenerative disease associated to the low oxidation of non-branched long chain saturated fatty acids (VLCFA; Very Long-Chain Fatty Acid) (Igarashi M., Schaumburg H.H., Powers J., Kishimoto Y., Kolodny E., Suzuki K., 1976. Fatty acid abnormality in adrenoleukodystrophy. Journal of Neurochemistry 26, 851-860), particularly hexacosanoic (C26:0) and tetracosanoic (C24:0) acid. Accumulation of VLCFA as cholesteryl esters in the plasma, adrenal cortex and white substance of the brain is due to a defect of the beta oxidation of the fatty acids at the peroxisomal level (Singh I., Moser A. E., Moser H. W., Kishimoto Y.,1984. Adrenoleukodystrophy: impaired oxidation of very long chain fatty acids in white blood cells, cultured skin fibroblasts and amniocytes. Pediatric Research 18, 286-290.). The most clear clinical manifestations are a progressive neurodegeneration process, demyelination, and hypogonadism as well as alopecia in some phenotypes.

The defective gene is in the disease map on gene Xq28 and encodes a peroxisomal transport protein ABCD1 (ATP-binding cassette) belonging to subfamily D, member 1, renamed ALDP. The disease is characterised by the presence of proteins homologous to ALDP belonging to the same family of membrane transporters on peroxisomes ALDR, PMP70, and P70R respectively encoded by genes *ABCD2, ABCD3* and *ABCD4.*

As of date, the treatment available for patients suffering from ALD is a therapy based on Lorenzo's Oil, a 4:1 mixture of glyceryl trioleate (oleic acid, 18:1) and glycerol trierucate (erucic acid,22:1) which, combined with a reduction of the saturated fatty acids in the diet, seems to normalize the levels of VLCFA (Rizzo W.B., Leshner R.T., Odone A., Dammann A., Craft D.A., 1989. Dietary erucic acid therapy for X-Adrenoleukodystrophy. Neurology,39,1415-1422) in patients.

The problem associated to this type of therapy lies in the fact that the main constituent: erucic acid, hardly passes through the blood-brain barrier (Uptake and metabolism of plasma-derived erucic acid by rat brain, 2006. Golovko and Murphy E.J.J.Lipid Res. 47, 1289-1297) and therefore it has a low effect on the nervous system. Furthermore, clinical studies wherein Lorenzo's Oil has been used showed a reduction of the VLCFAs but not an improvement in the neurodegenerative processes (Follow up of 89 asymptomatic patients with adrenoleukodystrophy treated with Lorenzo's Oil, 2005. Moser H.W., Raymond G.V. et al. Arch. Neurol. 62:1073-1080).

Conjugated linoleic acids (CLA) are C18 fatty acids containing two ethylenic unsaturations which, contrary to linoleic acid (18:2, n-6), are conjugated.

The most common naturally-occurring CLAs hitherto isolated are those respectively having double bonds in positions 6-8, 7-9, 8-10, 9-11, 10-12, 11-13, 12-14. Each of these acids may be in form of 4 geometric isomers and cis/cis isomer, trans/trans isomer, cis/trans isomer and trans/cis isomer respectively.

Therefore, 24 naturally occurring CLAs have been identified up to now.

Of these, the most abundant are the 9cis-11 trans-CLA and 10trans-12cis-CLA isomers.

The best source of CLA in nature is food of animal origin especially ruminant meat and milk as well as dairy products thereof. However, CLA may also be found in eggs and fish fat as well as sea animals. Low concentrations of CLA are also found in vegetable oils.

The best source of CLAs are dairy products containing more than 75% of 9cis-11trans, and 9trans-11 cis isomers. This type of acids has several therapeutic functions, among the main ones are an anticarcinogenic activity (see Banni S., Angioni E, Carta G, Mc. Ginley J, Thompson HJ, Barbano D: "Conjugated linoleic acid enriched butter alters mammary gland morphogenesis and reduces cancer risks in rats". J,. Nutr. (129) 2135-2142 (1999) mainly due to the abovementioned 9cis-11trans-CLA (see Lavillonieres F., Chajes V., Martin, J.C. Sebedio , J.L. Lhuillery , C.Bougnoux P., Dietary purified cis9, trans11-conjugated linoleic acid isomer has anticarcinogenic properties in chemically induced mammary tumors in rats. Nutrition and Cancer 2003 45 190-194,). 10trans-12cis CLA isomer has a considerable impact on the metabolism of fats and thus it is used in weight loss diets in that it reduces abdominal fat (see for example: Thom, E, Wadstein J, Gudmunsen O. Sep-Oct 2001 "Conjugated linoleic acid reduces body fat in healthy exercising humans" The Journal of Medical Research 29(5) 392-396), and for treating diabetes in that it reduces the absorption of glucose (see for example J.W. Ryder, C.P. Portocarro, ,X.M. Song, L. Cui, M. Yu, T. Combatsiaris, D. Galuska, D. and Barman, D.E. Baumn. D.M. Barbano, Mj Charron, J.R. Zierath and K.L. Houseknecht "Isomer Specific Antidiabetic Properties of Conjugated Linoleic acid" Diabetes (50)1149-1157 2001).

Experiments on animal models showed how CLA is capable of passing through the blood-brain barrier in that it is incorporated in the brain. "Incorporation and metabolism of c9-t11 and t 10-c12 conjugated linoleic acid (CLA) isomer in rat brain." Mauro Fa, Andrea Diana, Lina Cordeddu, Maria Paola Melis, Elisabetta Murru, Valeria Sogos, Sebastiano Banni. Biochimica et Biophysica Acta 1736 (2005) 61-66.

### SUMMARY OF THE INVENTION.

The Applicant has now discovered that the association of glycerol trioleate, glycerol trierucate with at least one conjugated linoleic acid is particularly effective in treating neurodegenerative diseases and adrenoleukodystrophy in particular.

As a matter of fact, as observed in the experimental part of the present description, alongside having a positive impact due to the reduction of VLCFA 26:0, the association of the abovementioned active ingredients lies in the fact that - contrary to erucic acid - the CLA passes through the blood-brain barrier in subjects suffering from neurodegenerative diseases and in particular the abovementioned adrenoleukodystrophy, thus having a positive effect also at a neurophysiological level.

In fact from the clinical trials described herein below an improvement in Somatosensory Evoked Potential was observed in ALD patients treated with the compositions of the invention, whereas this effect was absent in patients treated with Lorenzo's oil. Thus, medical dietary compositions containing GTO, GTE as active ingredient and at least one conjugated linoleic acid in combination with suitable diluents and/or excipients are an object of the present invention.

### DETAILED DESCRIPTION OF THE INVENTION

According to the present invention, the term "medical dietary composition" means a composition belonging to the category of "dietary foods for special medical purposes" which are a category of products for particular nutrition, regulated by the 89/398 EEC Community Directive, ratified in Italy by the Legislative Decree 111/92.

In other words, these are foods formulated based on reliable principles of medicine and nutritional science to effectively meet the particular nutritional needs of the subjects they are intended for according to generally accepted scientific data (Guidarelli, Copparoni and Scarpa: "Prodotti destinati ad una alimentazione particolare" Renzo Editore, pages 17-21, II° Edition). The weight ratio of glyceryl trioleate (oleic acid, 18:1) and glyceryl trierucate (erucic acid, 22:1) is preferably 4:1.

The GTO/GTE mixture and the conjugated linoleic acid are preferably present in the medical dietary composition according to the present invention at volumetric ratio comprised between 1:20 and 20:1. More preferably the GTO + GTE mixture and CLA are present in the dietary composition of the invention at weight ratio respectively comprised between 3:1 and 15:1. Even more preferably between 7:1 and 11:1. The presence of CLA in the composition of the invention has a further advantage, since it reduces the daily intake of erucic acid. In fact erucic acid is known to cause in the long run myocardial lipidosis ("Effects of Dietary Saturated Fat on Erucic Acid Induced Myocardial lipidosis in Rats" J.K.G. Kramer, F.D.Sauer, MS Wolynetz E.R. Farnworth and K.M. Johnston, Lipids vol. 27 no. 8. The total amount of said active ingredients is preferably comprised between 1 and 100% in weight on the total weight of the medical dietary composition.

As used herein, the term conjugated linoleic acid is used to indicate any linoleic acid having ethylenic unsaturations at any position from C2-C4 to C15-C17 hence 14 isomers. Given that each of these isomers may have 4 geometric isomers i.e. cis/trans, trans/cis, cis/ cis, trans/trans, the composition according to the present invention may contain at least one out of the 56 spatial isomers of the conjugated linoleic acid.

At least one conjugated linoleic acid having the ethylenic unsaturations in the positions comprised between C7-C9 to C12-C14 is preferably used for the objects of the present invention. Thus, the compositions subject of the present invention preferably contain at least one out of the 24 isomers wherein the ethylenic unsaturations are respectively present in C7-C9, C8-C10, C9-C11, C10-C12, C11-C13, C12-C14.

More preferably the conjugated acids subject of the present invention are the geometric isomers of the conjugate having the ethylenic unsaturations respectively at carbon 9 and at carbon 11 and the one with the ethylenic unsaturations at carbon 10 and at carbon 12 and their mixtures. Thus, the compositions subject of the present invention more preferably contain at least one of the eight geometric isomers having the ethylenic unsaturations at positions C9-C11, and C10-C12

Even more preferably they are selected from among cis/trans and trans/cis isomers of said conjugated acids and according to the most preferred embodiment they are a mixture of 9cis-11 trans CLA and 10trans-12cis CLA. Said mixture is present at weight ratios preferably comprised between 1:20 and 20:1, The medical dietary compositions according to the present invention are in oral, liquid oil or soft capsules form.

Assessment tests regarding the efficiency of the compositions subject of the present invention, provided for exemplifying purposes, are provided below.

### Mixture effectiveness test

### Mixture preparation

50 ml of a CLA mixture - c9, t11 and t10, c12 in 1:1 ratio - are added to 500 ml containing as the main component a of a GTO/GTE 4:1 mixture.

The formulation in the form of an oil thus obtained has therefore the following final composition expressed as percentage by weight
61.3% oleic acid,
15.0% erucic acid,
9.9% conjugated linoleic acid

### Experiment

5 female patients affected by X-ALD, heterozygote, is recruited. Diagnosis is carried out by means of genetic test and plasmatic dosage of VLCFAs.
Time 0: 10 ml of blood in EDTA are drawn from the patient. After centrifugation of the corpuscular part the plasma is frozen at -20°C.
Oral intake of the mixture, at amounts of 40 ml/die starts from time 0 and continues daily for 2 months.
Time 2 months: blood is drawn from the patient and the plasma is obtained as indicated in time 0.
The liquor is drawn and frozen at -20°C.

### Methods

Total lipids were extracted from the plasma and liquor according to Folch method (Folch J, Lees M, Sloane-Stanley GH. A simple method for the isolation and purification of total lipid from animal tissues. J. Biol. Chem. 1957;226:497-509); (Melis MP, Angioni E, Carta G, et al. Characterization of conjugated linoleic acid and its metabolites by RP-HPLC with diode array detector European Journal of Lipid Science & Technology 2001;103:617-621).

Free fatty acids were analysed through HPLC. The separation of CLA and its metabolites was carried out through Hewlett-Packard 1100 HPLC system (Hewlett-Packard, Palo Alto, CA). A C-18 Inertsil 5 ODS-2 Chrompack column (Chrompack International BV, Middleburg, The Netherlands), 5µm 150 x 4.6 mm was used, with a mobile phase of CH3CN/H2O/CH3COOH (70/30/0.12, v/v/v) at a flow rate of 1.5 ml/min (Melis MP, Angioni E, Carta G, et al. Characterization of conjugated linoleic acid and its metabolites by RP-HPLC with diode array detector. European Journal of Lipid Science & Technology 2001;103:617-621). The conjugated dienes were determined at 234nm.

### Results

**Tab.1. Levels of fatty acids (nmoles/ml) +/- S.D.**

| | 18:1 (oleic acid) | 22:1 (erucic acid) | 18:2 (CLA) | 26:0 | 26:0/22:0 |
|---|---|---|---|---|---|
| **Plasma** | | | | | |
| Time | 0 1917.96±490.95 | 5.73±1.87 | 10.43±2.4 | 88.73±34.08 | 1.14±0.39 |
| Time 2 | 2299.78±316.48 | 10.71±3.05 | 47.6±12.9 | 70.18±20.73 | 0.73±0,14 |

| **Liquor** | | | | | |
|---|---|---|---|---|---|
| Time 0 | 11.76±6.6 | ND | 0.005±0.003 | ND | |
| Time 2 | 15.4±7.5 | ND | 0.012±0.00,5 | ND | |

The levels of VLCFA 26:0 and ratio 26:0/22:0 had dropped considerably after 2 months of treatment.

After 2 months of treatment the levels of oleic acid, erucic acid and CLA, present in the mixture administered to the patient, had increased considerably, indicating good absorption of the components.

The liquor, drawn after 2 months of treatment does not contain erucic acid while CLA is present.

Such data indicates the passage of CLA through the blood-brain barrier; erucic acid does not pass through such barrier.

3 out five female carriers underwent also somatosensory evoked potential (SEP) evaluations according to the method described in "Neurophysiological abnormalities in adrenoleukodystrophy carriers- Evidence of different degrees of central nervous system involvement" D. Restuccia, V. Di Lazzaro, M. Valeriani, A. Oliviero, D. Le Pera, C. Colosimo, N. Burdi, M. Cappa, E. Bertini, A. Di Biase and P. Tonali. Brain (1997), 120 1139-1148. All 3 patients exhibited improved SEP values if compared to those obtained with patients affected by ALD and treated only with Lorenzo's oil and reported in "Neurophysiologic follow up of long term dietary treatment in adult onset adrenoleukodystrophy" D. Restuccia, V. Di Lazzaro, M. Valeriani, A. Oliviero, D. Le Pera, Barba C., M. Cappa, E. Bertini, A. Di Capua M. and P. Tonali, Neurology, volume 52(4), 10 March 1999, 810-816.

## Claims

1. Medical dietary compositions comprising an association of glyceryl trioleate (GTO), glyceryl trierucate (GTE) and an association of at least one conjugated linoleic acid as the active ingredient in combination with suitable excipients and/or diluents.

2. Compositions according to claim 1 wherein the GTO and the GTE are present at weight ratios of 4:1 with respect to each other.

3. Compositions according to any one of claims 1-2, wherein the GTO/GTE mixture and the conjugated linoleic acid are in volumetric ratios in the range between 1:20 and 20:1.

4. Compositions according to anyone of claims 1-3, wherein the GTO/GTE mixture and the conjugated linoleic acid are in weight ratio comprised between 3:1 and 15:1.

5. Compositions according to claim 4, wherein said weight ratio is comprised between 7:1 and 11:1.

6. Compositions according to any one of claims 1-5 wherein the total concentration of the active ingredient is in the range between 1 and 100 % by weight on the total weight of the composition.

7. Compositions according to any one of claims 1-6, comprising at least one conjugated linoleic acid selected among the 24 isomers wherein the ethylenic unsaturations are respectively present in C7-C9, C8-C10, C9-C11, C10-C12, C11-C13, C12-C14.

8. Compositions according to claim 7, comprising a mixture of 9cis-11 trans conjugated linoleic acid and 10 trans-12cis conjugated linoleic acid.

9. Compositions according to claim 8, wherein the weight ratio between 9 cis-11 trans conjugated linoleic acid and 10 trans-12 cis is in the range between 1:20 and 20:1.

10. Composition's according to any one of claims 1-9 for use in a method of treating neurodegenerative diseases.

11. Compositions according to claim 10, for use in a method of treating adrenoleukodystrophy.

## Patentansprüche

1. Medizinische Nahrungszusammensetzungen, umfassend eine Mischung aus Glycerintrioleat (GTO), Glycerintrierucat (GTE) und einer Mischung aus wenigstens einer konjugierten Linolsäure als Wirkstoff in Kombination mit geeigneten Hilfsstoffen und/oder Verdünnungsmitteln.

2. Zusammensetzungen nach Anspruch 1, wobei GTO und GTE in einem Gewichtsverhältnis von 4:1 in Bezug aufeinander vorliegen.

3. Zusammensetzungen nach einem der Ansprüche 1 bis 2, wobei die GTO-GTE-Mischung und die konjugierte Linolsäure in einem Volumenverhältnis im Bereich von 1:20 bis 20:1 vorliegen.

4. Zusammensetzungen nach einem der Ansprüche 1 bis 3, wobei die GTO-GTE-Mischung und die konjugierte Linolsäure in einem Gewichtsverhältnis von 3:1 bis 15:1 vorliegen.

5. Zusammensetzungen nach Anspruch 4, wobei das Gewichtsverhältnis zwischen 7:1 und 11:1 beträgt.

6. Zusammensetzungen nach einem der Ansprüche 1 bis 5, wobei die Gesamtkonzentration des Wirkstoffes im Bereich von 1 bis 100 Gewichtsprozent in Bezug zum Gesamtgewicht der Zusammensetzung beträgt.

7. Zusammensetzungen nach einem der Ansprüche 1 bis 6, umfassend wenigstens eine konjugierte Linolsäure, die aus den 24 Isomeren ausgewählt ist, wobei die ethylenischen Ungesättigheiten in C7-C9, C8-C10, C9-C11, C10-C12, C11-C13 und C12-C14 vorliegen.

8. Zusammensetzungen nach Anspruch 7, umfassend eine Mischung aus 9-cis-11-trans-konjugierter Linolsäure und 10-trans-12-cis-konjugierter Linolsäure.

9. Zusammensetzungen nach Anspruch 8, wobei das Gewichtsverhältnis zwischen 9-cis-11-trans-konjugierter Linolsäure und 10-trans-12-cis im Bereich von 1:20 bis 20:1 liegt.

10. Zusammensetzungen nach einem der Ansprüche 1 bis 9 zur Verwendung in einem Verfahren zum Behandeln von neurodegenerativen Erkrankungen.

11. Zusammensetzungen nach Anspruch 10 zur Verwendung in einem Verfahren zum Behandeln von Adrenoleukodystrophie.

## Revendications

1. Compositions diététiques médicales comprenant une association de glycéryl trioléate (GTO), glycéryl triérucate (GTE) et une association d'au moins un acide linoléique conjugué comme ingrédient actif en combinaison avec des excipients et/ou diluants appropriés.

2. Compositions selon la revendication 1 où le GTO et le GTE sont présents avec des rapports de poids de 4 : 1 par rapport à l'autre.

3. Compositions selon l'une quelconque des revendications 1-2 où le mélange GTO/GTE et l'acide linoléique conjugué sont dans des rapports volumétriques dans la plage entre 1 : 20 and 20 : 1.

4. Compositions selon l'une quelconque des revendications 1-3 où le mélange GTO/GTE et l'acide linoléique conjugué sont dans un rapport de poids compris entre 3:1 and 15:1.

5. Compositions selon la revendication 4, où ledit rapport de poids est compris entre 7 : 1 et 11 : 1.

6. Compositions selon l'une quelconque des revendications 1-5 où la concentration totale de l'ingrédient actif se trouve dans la plage entre 1 et 100% en poids sur le poids total de la composition.

7. Compositions selon l'une quelconque des revendications 1-6, comprenant au moins un acide linoléique conjugué sélectionné parmi les 24 isomères où les instaurations éthyléniques sont respectivement présentes dans C7-C9, C8-C10, C9-C11, C10-C12, C11-C13, C12-C14.

8. Compositions selon la revendication 7, comprenant un mélange d'acide linoléique conjugué 9 cis-11 trans et d'acide linoléique conjugué 10 trans-12 cis.

9. Compositions selon la revendication 8 où le rapport de poids entre l'acide linoléique conjugué 9 cis-11 trans et le 10 trans-12 cis se trouve dans la plage entre 1:20 and 20:1.

10. Compositions selon l'une quelconque des revendications 1-9 pour une utilisation dans un procédé de traitement des maladies neurodégénératives.

11. Compositions selon la revendication 10, pour une utilisation dans un procédé de traitement l'adrénoleucodystrophie.
